# EUROPÄISCHE PATENTSCHRIFT

(11) **EP 1 035 872 B1**
(45) Veröffentlichungstag und Bekanntmachung des Hinweises auf die Patenterteilung: **19.02.2003**
(21) Anmeldenummer: 98965234.2
(22) Anmeldetag: 07.12.1998
(51) Int. Cl.: A61L 2/00

(54) **UV-DESINFEKTIONSEINRICHTUNG**
UV DISINFECTION DEVICE
DISPOSITIF DE DESINFECTION PAR ULTRAVIOLETS

(30) Priorität: 06.12.1997 DE 29721608 U
(43) Veröffentlichungstag der Anmeldung: 20.09.2000
(73) Patentinhaber: a.c.k. aqua concept GmbH Karlsruhe, 76189 Karlsruhe (DE)
(72) Erfinder: WECKENMANN, Jürgen, D-68163 Mannheim (DE); SÖRENSEN, Martin, D-76359 Marxzell (DE)
(74) Vertreter: Patentanwälte Ruff, Wilhelm, Beier, Dauster & Partner
(86) Internationale Anmeldenummer: EP9807926
(87) Internationale Veröffentlichungsnummer: WO99029351

(56) Entgegenhaltungen:
- DE-A- 4 220 088
- US-A- 5 440 131

## Beschreibung

Die Erfindung betrifft eine UV-Desinfektionseinrichtung für einen Behälter und eine darin lagernde Flüssigkeit, insbesondere zur Reduzierung von Mikroorganismen, die auf den Behälterinnenwänden und in der Flüssigkeit angesiedelt sind.

Eine derartige Reinigungseinrichtung wird üblicherweise zur chemikalienfreien Desinfektion von Flüssigkeiten eingesetzt, die in Behältern gelagert sind. Ein besonderes Problem stellen dabei Flüssigkeitsbehälter dar, deren Flüssigkeitsspiegel betriebsbedingt steigt und fällt.

Herkömmliche Desinfektionseinrichtungen, die mit Hilfe von UV-Licht arbeiten, liegen entweder am Behältergrund oder tauchen am Kabel oder einer Vorrichtung hängend in die Flüssigkeit ein. Solche Systeme sind nicht geeignet, sowohl die Flüssigkeit als auch die feuchten und oberhalb des Flüssigkeitsspiegels befindlichen Behälterwände zu desinfizieren (und diese Aufgabe auch bei wechselndem Behälterfüllstand zu erfüllen). Gerade die Strahlung der gänzlich in die Flüssigkeit eintauchenden Desinfektionseinrichtungen erfährt an weiten Bereichen des Flüssigkeitsspiegels Totalreflexion in die Flüssigkeit zurück, so dass die zu Verkeimung neigenden Behälterwände nicht oder nur unzureichend bestrahlt werden. Darüber hinaus sind die herkömmlichen Desinfektionseinrichtungen nicht geeignet, sich unter Ausschluss der Totalreflexion an den wechselnden Füllstand im Behälter anzupassen. Ein weiterer Nachteil herkömmlicher Desinfektionseinrichtungen besteht darin, dass die Vorrichtungen zur Strahlerhalterung und die Strahlerenden Schatten an immer die gleichen Bereiche der Behälterumschließungen werfen, weil diese Einrichtungen relativ zum Behälter starr angeordnet sind.

Die DE-A-42 20 088 zeigt eine Vorrichtung zur Desinfektion von Wasser im offenen Gerinne unter Verwendung von UV-Lampen. Dabei sind die UV-Lampen in einem schwimmfähigen Rahmen derart gelagert, dass sie sich um eine Achse im Boden des Gerinnes drehen können. Durch diese Konstruktion soll unter anderem gewährleistet werden, dass die UV-Lampen bzw. deren Schutzrohre während des Betriebs immer vollständig mit der zu bestrahlenden Flüssigkeit bedeckt sind.

### Aufgabe und Lösung

Es ist Aufgabe der Erfindung, die genannten Nachteile des Standes der Technik zu vermeiden und eine Desinfektion der Flüssigkeit sowie der-feuchten Behälterinnenwände zu verbessern.

Diese Aufgabe wird durch die Einrichtung mit den in Anspruch 1 aufgeführten Merkmalen gelöst. Bevorzugte Ausführungsformen sind in den abhängigen Ansprüchen 2 bis 15 dargestellt. Der Wortlaut sämtlicher Ansprüche wird hiermit durch Bezugnahme zum Inhalt dieser Beschreibung gemacht.

Mit der Erfindung wird erreicht, dass die UV-Desinfektionseinrichtung auf dem Flüssigkeitsspiegel schwimmt. Ein solches System hebt und senkt sich mit dem Flüssigkeitsspiegel und ist deshalb besonders an den kritischen Stellen des Obergangs von der Flüssigkeit zum Gasraum aktiv, ein Bereich, dessen Anfälligkeit zur Veralgung und Verkeimung besonders hoch ist. Die freie Beweglichkeit der erfindungsgemäßen Einrichtung um die Hochachse gewährleistet dabei, dass alle Bereiche der Behälterumschließungen bestrahlt werden, weil mögliche Schatten oder Bereiche minimaler Bestrahlung an den Behälterwänden entlangwandern und sich im zeitlichen Mittel ausgleichen.

Vorteilhaft ist es, wenn der Schwimmer ein zentraler Schwimmer ist. Wenigstens ein UV-Strahler kann röhrenförmig sein, wobei vorzugsweise alle UV-Strahler röhrenförmig sind. Wenigstens ein UV-Strahler kann parallel zur Ebene, insbesondere in der Ebene, des Flüssigkeitsspiegels verlaufen, vorzugsweise mehrere UV-strahler. Vorteilhaft verläuft wenigstens ein UV-strahler in einem Winkel von 0° bis 90° zur Ebene des Flüssigkeitsspiegels. Es kann vorgesehen sein, daß wenigstens ein UV-strahler zumindest teilweise in die Flüssigkeit eintaucht, insbesondere etwa mit der Hälfte seiner Ausdehnung, wobei er vorzugsweise die Ebene des Flüssigkeitsspiegels durchstößt. Bevorzugt tauchen mehrere bzw. eine überwiegende Zahl von UV-Strahlern in die Flüssigkeit zumindest teilweise ein. So werden die Flüssigkeit und der Raum oberhalb der Flüssigkeit gleichzeitig bestrahlt. Wenigstens ein UV-Strahler kann vollständig in die Flüssigkeit eingetaucht sein, wobei er insbesondere senkrecht zur Ebene des Flüssigkeitsspiegels verläuft.

Eine vorteilhafte Ausgestaltung der erfindungsgemäßen Einrichtung sieht vor, daß an einem zentralen Schwimmer in der Ebene oder parallel zur Ebene des Flüssigkeitsspiegels angeordnete Stäbe, Drähte oder dergleichen, welche vorzugsweise elastisch sind, angebracht sind. Sie zentrieren die UV-Desinfektionseinrichtung in der Behältermitte ohne ihre Beweglichkeit in Richtung der Hochachse einzuschränken. Die Längen der elastischen Stäbe können derart abgestimmt sein, daß der Schwimmer an einer bestimmten Position in der Ebene des Flüssigkeitsspiegels fixiert wird, insbesondere in der Mitte des Behälters zentriert wird.

Eine weitere Möglichkeit, den Schwimmer in der Behältermitte zu zentrieren ohne seine Beweglichkeit in Richtung der Hochachse einzuschränken, kann vorsehen, daß der Schwimmer an einer an dem Behälterboden oder der Behälterdecke angebrachten und senkrecht zum Flüssigkeitsspiegel verlaufenden Stange beweglich angeordnet ist und entsprechend dem Flüssigkeitsstand nach oben oder unten gleitet. Die Stange dient als Fixierung für den Schwimmer, um mit dem Flüssigkeitsspiegel zu steigen oder zu fallen und sich um einen bestimmten Winkel um die Hochachse zu drehen. Eine vorteilhafte Weiterbildung der Erfindung kann vorsehen, daß das System so austariert ist, daß wenigstens ein in der Ebene des Flüssigkeitsspiegels angebrachter UV-Strahler zu einem wesentlichen Teil seines Umfanges (beispielsweise 70 %), jedoch nicht vollständig, in die Flüssigkeit eintaucht. Damit wird erreicht, daß sowohl die Flüssigkeit als auch der Gasraum oberhalb der Flüssigkeit mit UV-Licht beaufschlagt wird.

In dem Schwimmer kann ein Neigungsschalter untergebracht sein, mit dessen Hilfe der wenigstens eine UV-Strahler bei Überschreiten einer bestimmten Neigungs-Grenzlage durch den Schwimmer abschaltbar ist. Wenn der Flüssigkeitsspiegel auf ein bestimmtes Niveau abfällt, neigt sich der Schwimmer der erfindungsgemäßen Desinfektionseinrichtung zur Seite und ein in ihm integrierter Neigungsschalter unterbricht die Stromversorgung. So kann detektiert werden, ob der Behälter leer ist und infolgedessen der wenigstens eine Strahler abgeschaltet werden soll. Weiters kann an dem Schwimmer ein Sensor zur Messung der in dem Behälter vorhandenen Flüssigkeit angebracht sein, insbesondere an einer der Außenflächen außerhalb des Schwimmers. Dieser detektiert den Flüssigkeitsmangel und schaltet ggf. die Stromversorgung des wenigstens einen UV-Strahlers ab.

Vorteilhaft können zum Betrieb des wenigstens einen UV-Strahlers notwendige Vorschaltgeräte in dem Schwimmer untergebracht sein, wobei sie über ein elektrisches Kabel mit einer externen Spannungsquelle verbunden sind. Hier können sie zusätzlich als Ballast für dessen stabile Schwimmlage dienen. Wenn ihre Größe oder ihre Masse den Einbau in den Schwimmer untersagen, können die Vorschaltgeräte außerhalb des Schwimmers untergebracht sein, wobei der UV-Strahler über ein elektrisches Kabel mit der entsprechend transformierten Spannung versorgt werden kann.

### Kurzbeschreibung der Zeichnungen

Die Figuren erläutern beispielhaft den Aufbau einer UV-Desinfektionseinrichtung. In den Figuren zeigen:
- Fig. 1 und 2:: UV-Desinfektionseinrichtungen im Einsatz in einem liegenden und in einem stehenden Behälter.
- Fig. 3 und 4:: Draufsicht und Ansicht einer UV-Desinfektionseinrichtung und
- Fig. 5:: Ansicht einer UV-Desinfektionseinrichtung mit schräg durch den Flüssigkeitsspiegel hindurchstoßenden UV-Strahlern.

### Detaillierte Beschreibung der Ausführungsbeispiele

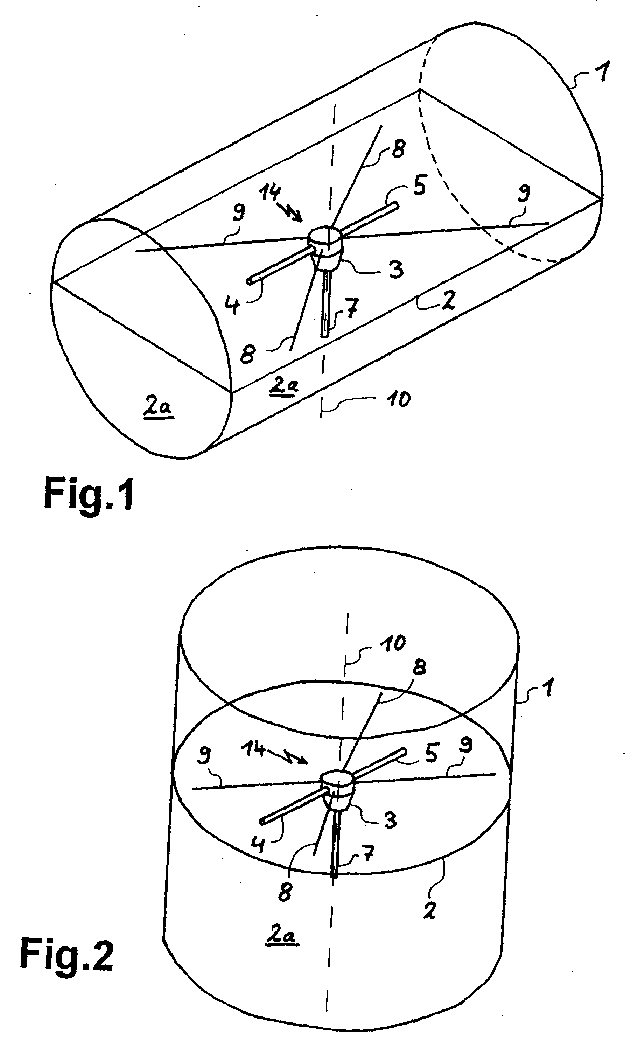

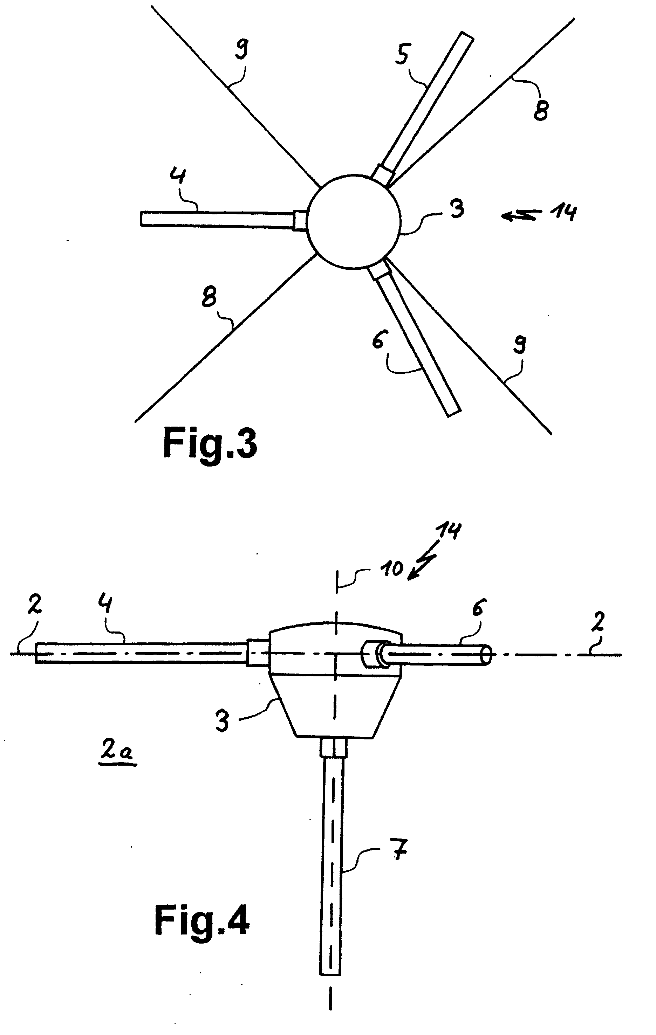

Fig. 1 zeigt ein Beispiel einer UV-Desinfektionseinrichtung 14 in einem liegenden Behälter 1, Fig. 2 in einem stehenden Behälter 1, wobei die erfindungsgemäße Einrichtung am Beispiel des stehenden Behälters erklärt werden soll. Der Behälter 1 ist mit Flüssigkeit 2a gefüllt und auf dem Flüssigkeitsspiegel 2 schwimmt die erfindungsgemäße UV-Desinfektionseinrichtung 14. Sie besteht aus dem Schwimmer 3, den UV-Strahlern 4, 5 und 7 und den (beiden) elastischen Stäben 8 und 9. Die elastischen Stäbe 8 und 9 zentrieren die Desinfektionseinrichtung 14 in der Behältermitte, ohne ihre Beweglichkeit in Richtung der Hochachse 10 der Desinfektionseinrichtung 14 einzuschränken, so daß keine fixen Schatten an den Behälterinnenwänden entstehen. Die horizontalen Strahler 4 und 5 tauchen zu einem gewissen Teil in die Flüssigkeit 2a ein (siehe auch Fig. 4) und desinfizieren auf diese Weise die Flüssigkeit und die oberhalb des Flüssigkeitsspiegels 2 befindlichen und im allgemeinen feuchten Behälterwände.

In Fig. 3 ist ein weiteres Beispiel einer UV-Desinfektionseinrichtung 14 in der Draufsicht, in Fig. 4 in der Ansicht dargestellt. Die UV-Desinfektionseinrichtung 14 ist in der Ebene des Flüssigkeitsspiegels 2 mit drei UV-Strahlern 4, 5 und 6 ausgerüstet, die in Fig. 3 zu sehen sind, in der Ansicht in Fig. 4 jedoch nur die UV-strahler 4 und 6. In Fig. 4 ist der UV-Strahler 7 zu sehen, der vollständig und senkrecht in die Flüssigkeit 2a eintaucht. Des weiteren sind in Fig. 3 die Stäbe 8 und 9 dargestellt.

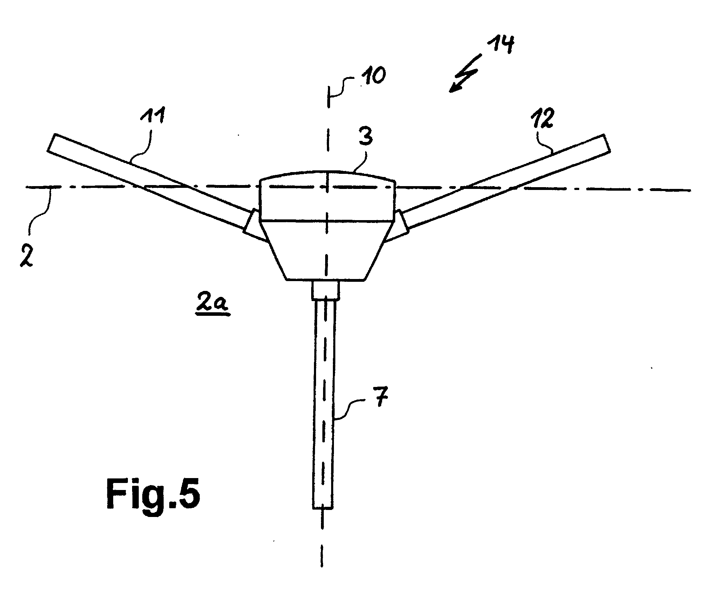

Fig. 5 zeigt eine weitere mögliche Ausbildung einer erfindungsgemäßem UV-Desinfektionseinrichtung 14 mit zwei UV-Strahlern 11 und 12, die den Flüssigkeitsspiegel 2 schräg durchstoßen. Zu sehen ist weiterhin ein UV-Strahler 7, der senkrecht zur Ebene des Flüssigkeitsspiegels 2 ganz in die Flüssigkeit 2a eintaucht.

## Patentansprüche

1. UV-Desinfektionseinrichtung (14) für einen Behälter (1) und darin lagernde Flüssigkeit (2a), insbesondere zur Reduzierung von auf den Behälterinnenwänden und in der Flüssigkeit angesiedelten Mikroorganismen, wobei die Einrichtung (14) mit Hilfe eines um die Hochachse (10) der Einrichtung frei beweglichen Schwimmers (3) schwimmfähig ausgebildet ist und an dem Schwimmer (3) mindestens ein UV-Strahler (4, 5, 6, 7, 11, 12) derart angeordnet ist, dass eine gleichzeitige Bestrahlung der Flüssigkeit (2a) und von oberhalb des Flüssigkeitsspiegels (2) befindlichen Behälterinnenwänden erfolgt.

2. UV-Desinfektionseinrichtung nach Anspruch 1, **dadurch gekennzeichnet, dass** der Schwimmer (3) ein zentraler Schwimmer ist.

3. UV-Desinfektionseinrichtung nach Anspruch 1 oder 2, **dadurch gekennzeichnet, dass** der UV-Strahler (4, 5, 6, 7, 11, 12) röhrenförmig ausgebildet ist.

4. UV-Desinfektionseinrichtung nach einem der vorhergehenden Ansprüche, **dadurch gekennzeichnet, dass** mindestens ein UV-Strahler, vorzugsweise drei UV-Strahler (4, 5, 6) parallel zur Ebene, insbesondere in der Ebene des Flüssigkeitsspiegels (2) verlaufen.

5. UV-Desinfektionseinrichtung nach einem der vorhergehenden Ansprüche, **dadurch gekennzeichnet, dass** mindestens ein UV-Strahler (7, 11, 12) in einem Winkel bis zu 90° zur Ebene des Flüssigkeitsspiegels (2) verläuft.

6. UV-Desinfektionseinrichtung nach einem der vorhergehenden Ansprüche, **dadurch gekennzeichnet, dass** mindestens ein UV-Strahler (4, 5, 6, 11, 12) teilweise in die Flüssigkeit (2a) eintaucht, insbesondere etwa zur Hälfte, wobei vorzugsweise der UV-Strahler (11, 12) die Ebene des Flüssigkeitsspiegels (2) durchstößt.

7. UV-Desinfektionseinrichtung nach einem der vorhergehenden Ansprüche, **dadurch gekennzeichnet, dass** mindestens ein UV-Strahler (7) vollständig in die Flüssigkeit (2a) eingetaucht ist, wobei er insbesondere senkrecht zur Ebene des Flüssigkeitsspiegels (2) verlauft.

8. UV-Desinfektionseinrichtung nach einem der vorhergehenden Ansprüche, **dadurch gekennzeichnet, dass** an dem Schwimmer (3) vorzugsweise elastische Stäbe (8, 9) parallel zur Ebene des Flüssigkeitsspiegels (2) angeordnet sind, insbesondere in der Ebene des Flüssigkeitsspiegels.

9. UV-Desinfektionseinrichtung nach Anspruch 8, **dadurch gekennzeichnet, dass** die Längen der elastischen Stäbe (8, 9) abgestimmt sind, um den Schwimmer (3) an einer bestimmten Position in der Ebene des Flüssigkeitsspiegels (2) zu fixieren, insbesondere in der Mitte des Behälters (1) zu zentrieren.

10. UV-Desinfektionseinrichtung nach einem der vorhergehenden Ansprüche, **dadurch gekennzeichnet, dass** der Schwimmer (3) an einer an dem Behälterboden oder der Behälterdecke angebrachten und senkrecht zum Flüssigkeitssplegel (2) verlaufenden Stange oder dergleichen beweglich derart angeordnet ist, dass er entsprechend dem Flüssigkeitsstand nach oben oder unten gleiten kann, wobei vorzugsweise der Schwimmer (3) in einem gewissen Winkel um die Stange oder dergleichen drehbar ist.

11. UV-Desinfektionseinrichtung nach Anspruch 4, **dadurch gekennzeichnet, dass** die UV-Desinfektionseinrichtung (14) austariert ist, um den mindestens einen in der Ebene des Flüssigkeitsspiegels (2) angebrachten UV-Strahler (4, 5, 6) zu einem Teil, insbesondere einem wesentlichen Teil seines Umfangs in die Flüssigkeit (2a) einzutauchen, wobei der Rest seines Umfangs über den Flüssigkeitsspiegel ragt.

12. UV-Desinfektionseinrichtung nach einem der vorhergehenden Ansprüche, **dadurch gekennzeichnet, dass** in dem Schwimmer (3) ein Neigungsschalter untergebracht ist, mit dessen Hilfe der mindestens eine UV-Strahler (4, 5, 6, 7, 11, 12) bei Überschreiten einer bestimmten Neigungs-Grenzlage des Schwimmers (3) abschaltbar ist.

13. UV-Desinfektionseinrichtung nach einem der vorhergehenden Ansprüche, **dadurch gekennzeichnet, dass** an dem Schwimmer (3) ein Sensor zur Messung der in dem Behälter (1) vorhandenen Flüssigkeit (2a) angebracht ist.

14. UV-Desinfektionseinrichtung nach einem der vorhergehenden Ansprüche, **dadurch gekennzeichnet, dass** zum Betrieb des mindestens einen UV-Strahlers (4, 5, 6, 7, 11, 12) notwendige Vorschattgeräte in dem Schwimmer (3) untergebracht sind, wobei diese über ein elektrisches Kabel mit einer externen Spannungsquelle verbunden sind.

15. UV-Desinfektionseinrichtung nach einem der Ansprüche 1 bis 13, **dadurch gekennzeichnet, dass** zum Betrieb des mindestens einen UV-Strahlers (4, 5, 6, 7, 11, 12) notwendige Vorschaltgeräte außerhalb des Schwimmers (3) untergebracht sind und der UV-Strahler über ein elektrisches Kabel mit der entsprechend transformierten Spannung versorgt wird.

## Claims

1. UV disinfection device (14) for a container (1) and the liquid (2a) stored therein, particularly for reducing microorganisms which have colonized the container inner walls and the liquid, the device (14) being constructed in floatable manner with the aid of a float (3) freely movable about the vertical axis (10) of the device and on the float (3) is provided at least one UV radiator (4, 5, 6, 7, 11, 12) in such a way that there is a simultaneous irradiation of the liquid (2a) and the container inner walls above the liquid level (2).

2. UV disinfection device according to claim 1, **characterized in that** the float (3) is a central float.

3. UV disinfection device according to claim 1 or 2, **characterized in that** the UV radiator (4, 5, 6, 7, 11, 12) has a tubular construction.

4. UV disinfection device according to one of the preceding claims, **characterized in that** at least one and preferably three UV radiators (4, 5, 6) are parallel to the plane and are in particular in the plane of the liquid level (2).

5. UV disinfection device according to one of the preceding claims, **characterized in that** at least one UV radiator (7, 11, 12) is at an angle of up to 90ø to the plane of the liquid level (2).

6. UV disinfection device according to one of the preceding claims, **characterized in that** at least one UV radiator (4, 5, 6, 11, 12) is partly and in particular approximately half immersed in the liquid (2a) and preferably the UV radiator (11, 12) passes through the plane of the liquid level (2).

7. UV disinfection device according to one of the preceding claims, **characterized in that** at least one UV radiator (7) is completely immersed in the liquid (2a) and in particular is perpendicular to the plane of the liquid level (2).

8. UV disinfection device according to one of the preceding claims, **characterized in that** on the float (3) and parallel to the plane of the liquid level (2) are provided preferably elastic rods (8, 9), which are particularly located in the liquid level plane.

9. UV disinfection device according to claim 8, **characterized in that** the lengths of the elastic rods (8, 9) are matched so as to fix the float (3) at a specific position in the plane of the liquid level (2), particularly centring in the centre of the container (1).

10. UV disinfection device according to one of the preceding claims, **characterized in that** the float (3) is movably arranged on a bar or the like fitted to the container bottom or top and running perpendicular to the liquid level (2) in such a way that it can slide upwards or downwards in accordance with the liquid level and preferably the float (3) is rotatable by a certain angle about the bar or the like.

11. UV disinfection device according to claim 4, **characterized in that** the UV disinfection device (14) is tared, so as to in part and in particular by a significant part of the circumference to immerse the at least one UV radiator (4, 5, 6) fitted in the plane of the liquid level (2), the remainder of its circumference projecting above the liquid level.

12. UV disinfection device according to one of the preceding claims, **characterized in that** in the float (3) is housed an inclination switch with the aid of which the at least one UV radiator (4, 5, 6, 7, 11, 12) can be switched off when the float (3) passes beyond a specific inclination limit position.

13. UV disinfection device according to one of the preceding claims, **characterized in that** a sensor for measuring the liquid (2a) present in the container (1) is fitted to the float (3).

14. UV disinfection device according to one of the preceding claims, **characterized in that** ballasts necessary for the operation of the at least one UV radiator (4, 5, 6, 7, 11, 12) are housed in the float (3) and are connected by an electric cable to an external voltage source.

15. UV disinfection device according to one of the claims 1 to 13, **characterized in that** the ballasts necessary for the operation of the at least one UV radiator (4, 5, 6, 7, 11, 12) are housed outside the float (3) and the UV radiator is supplied by an electric cable with the correspondingly transformed voltage.

## Revendications

1. Dispositif de désinfection aux UV (14) pour un récipient (1) et le liquide stocké (2a) à l'intérieur, en particulier pour la réduction de microorganismes logés sur les parois du récipient et dans le liquide, le dispositif (14) étant réalisé flottant à l'aide d'un flotteur (3) librement mobile autour de l'axe vertical (10) du dispositif et sur le flotteur (3) étant disposé au moins un radiateur UV (4, 5, 6, 7, 11, 12) de sorte qu'une irradiation simultanée du liquide (2a) et des parois internes du récipient se trouvant au-dessus du niveau de liquide (2).

2. Dispositif de désinfection aux UV selon la revendication 1, **caractérisé en ce que** le flotteur (3) est un flotteur central.

3. Dispositif de désinfection aux UV selon la revendication 1 ou 2, **caractérisé en ce que** le radiateur UV (4, 5, 6, 7, 11, 12) est tubulaire.

4. Dispositif de désinfection aux UV selon l'une des revendications précédentes, **caractérisé en ce qu'**au moins un radiateur UV, de préférence trois radiateurs UV (4, 5, 6) s'étendent parallèlement au plan, en particulier dans le plan du niveau de liquide (2).

5. Dispositif de désinfection aux UV selon l'une des revendications précédentes, **caractérisé en ce qu'**au moins un radiateur UV (7, 11, 12) s'étend dans un angle maximal de 90° par rapport au plan du niveau de liquide (2).

6. Dispositif de désinfection aux UV selon l'une des revendications précédentes, **caractérisé en ce qu'**au moins un radiateur UV (4, 5, 6, 7, 11, 12) est plongé partiellement dans le liquide (2a), en particulier à peu prés à moitié, de préférence le radiateur UV (11, 12) traversant peu près à moitié, de préférence le radiateur UV (11, 12) traversant le plan du niveau de liquide (2).

7. Dispositif de désinfection aux UV selon l'une des revendications précédentes **caractérisé en ce qu'**au moins un radiateur UV (7) est plongé complètement dans le liquide (2a), lequel s'étend en particulier perpendiculairement au plan du niveau de liquide (2).

8. Dispositif de désinfection aux UV selon l'une des revendications précédentes **caractérisé en ce que** sur le flotteur (3), des barres (8, 9) de préférence élastiques sont disposées parallèlement au plan du niveau de liquide (2), en particulier dans le plan du niveau de liquide.

9. Dispositif de désinfection aux UV selon la revendication 8 **caractérisé en ce que** les longueurs des barres élastiques (8, 9) sont ajustées pour fixer le flotteur (3) à une position définie dans le plan du niveau de liquide (2), en particulier au milieu du récipient (1).

10. Dispositif de désinfection aux UV selon l'une des revendications précédentes **caractérisé en ce que** le flotteur (3) est disposé de manière mobile sur une tige ou similaire ménagée sur le fond du récipient ou le dessus du récipient et s'étend perpendiculairement au niveau de liquide (2) de sorte que le flotteur glisse vers le haut ou vers le bas par rapport au niveau de liquide, de préférence le flotteur (3) étant rotatif autour de la tige ou similaire selon un certain angle.

11. Dispositif de désinfection aux UV selon la revendication 4, **caractérisé en ce que** le dispositif de désinfection aux UV (14) est taré, pour plonger au moins un radiateur UV (4, 5, 6) ménagé dans le plan du niveau de liquide (2), en partie, en particulier une partie essentielle de sa périphérie dans le liquide (2a), le reste de sa périphérie dépassant du niveau de liquide.

12. Dispositif de désinfection aux UV selon l'une des revendications précédentes, **caractérisé en ce que** dans le flotteur (3), un interrupteur d'inclinaison étant logé, à l'aide duquel au moins un radiateur UV (4, 5, 6, 7, 11, 12) étant déconnectable lors du dépassement d'une certaine position limite d'inclinaison du flotteur (3).

13. Dispositif de désinfection aux UV selon l'une des revendications précédentes, **caractérisé en ce que** sur le flotteur (3) est disposé un capteur pour la mesure du liquide (2a) se trouvant dans le récipient (1).

14. Dispositif de désinfection aux UV selon l'une des revendications précédentes, **caractérisé en ce que** pour le fonctionnement d'au moins un radiateur UV (4, 5, 6, 7, 11, 12), des ballasts nécessaires sont logés dans le flotteur (3), ces derniers étant reliés par un câble électrique à une source externe de tension.

15. Dispositif de désinfection aux UV selon l'une des revendications 1 à 13, **caractérisé en ce que** pour le fonctionnement d'au moins un radiateur UV (4, 5, 6, 7, 11, 12), des ballasts nécessaires sont logés dans le flotteur (3), ces derniers étant reliés par un câble électrique à la tension transformée correspondante.
